# EUROPEAN PATENT APPLICATION

(11) **EP 4 647 032 A1**
(43) Date of publication of application: **12.11.2025**
(21) Application number: 23923002.2
(22) Date of filing: 03.03.2023
(51) Int. Cl.: A61B 90/50, A61B 17/00

(54) **HOLDING CONTROLLER**

(30) Priority: 02.03.2023 KR 20230027815
(71) Applicant: Go, Moo-Saeng, Busan 48060 (KR)
(72) Inventor: Go, Moo-Saeng, Busan 48060 (KR)
(74) Representative: Murgitroyd & Company
(86) International application number: PCT/KR2023/002952
(87) International publication number: WO 2024/172200

(57) **Abstract**

The present invention relates to a holding controller and, in particular, to a holding controller that can adjust a fixing force for holding an object to be fixed such as a medical instrument, thus making it easy to change the position and angle of the object to be fixed in various situations, the holding controller comprising a holding body that has, provided in the front end thereof, a ball portion of a ball joint connected to an object to be fixed and can control the movement of the ball portion, wherein the holding body comprises therein: a fixed pressing member which can be in contact with the ball portion; an elastic member which transmits force to the fixed pressing member forward via the elastic force; and a fixed control means which contracts the elastic member backward or extends the elastic member forward.

## Description

### Technical Field

The present disclosure relates to a holding controller and, one particular implementation relates to a holding controller capable of adjusting a fixing force for holding an object to be fixed such as a medical device.

### Background Art

A variety of medical devices, such as endoscopes, drills, suctions, mirrors, and the like are commonly used to perform medical procedures in hospitals. These medical devices are mainly used by the user performing the treatment by holding them directly with his or her own hands. However, since it is not possible to grasp all the necessary medical devices, the user may receive help from an assistant or additionally use auxiliary devices that can fix the medical devices in a specific location.

However, when assisted by an assistant, it is difficult for the user performing the treatment to fix the medical device in the desired position. When using an assistive device, the medical device can be fixed in a specific position, but it is inconvenient because it can only be fixed and released: the angle of the medical device cannot be continuously changed during the treatment process; or the fixing force for holding the medical device cannot be adjusted.

### Detailed Description of the Invention

### Technical Goals

An aspect is to solve the above problems and provides a holding controller capable of adjusting a fixing force for holding an object to be fixed such as a medical device or the like, thereby facilitating altering the position and angle of the object to be fixed in various situations.

### Technical solutions

According to an aspect, there is provided a holding controller including a holding body having a ball part of a ball joint coupled to an object to be fixed, provided inside a front end, and configured to control a movement of the ball part, in which the holding body includes a fixing pressurization member configured to come into contact with the ball part, an elastic member configured to transmit force forward to the fixing pressurization member via elastic force, and a fixing controller configured to contract the elastic member backward or extend the elastic member forward.

The fixing controller may be configured to axially rotate about an axis oriented in a forward and backward direction, and an extent to which the fixing pressurization member pressurizes the ball part may change depending on contraction or extension of the elastic member according to the direction of axial rotation of the fixing controller.

The fixing controller may include a guard part configured to move forward and backward within the fixing pressurization member and contract the elastic member and a control rod extending rearward from the guard part to penetrate the elastic member and being screw-coupled to a rear end of the holding body.

The elastic member may be formed of a compression spring, and an outer end of the guard part may be positioned between an outer diameter and an inner diameter of the elastic member.

The holding controller may further include an adjustment guide plate provided at a front end of the elastic member on which a rear end of the fixing pressurization member and the guard part abut.

The holding body may further include an auxiliary elastic member having elasticity and provided in front of the fixing controller inside the fixing pressurization member.

### Effects of the Invention

According to the example embodiments of the present disclosure, since the fixing pressurization member fixes or releases the ball part as the elastic member is extended or contracted by the fixing controller, it is possible to firmly fix the object to be fixed or freely move the fixed object. In addition, the fixing force of the ball part can be adjusted by fine-tuning the fixing controller while the elastic member is maximally extended, so that the fixing force of holding the fixed object such as a medical device can be adjusted, and the position and angle of the fixed object can be easily altered in various situations.

### Brief Description of Drawings

FIG. 1 is a perspective view showing a holding controller of the present disclosure according to an example embodiment.
FIG. 2 is an exploded view showing a holding controller of the present disclosure according to an example embodiment.
FIG. 3 is a perspective view showing the internal structure of a holding controller of the present disclosure according to an example embodiment.
FIG. 4 is a side cross-sectional view showing the internal structure of a holding controller of the present disclosure according to an example embodiment.
FIG. 5 shows side cross-sectional views illustrating the states in which a ball part applied to a holding controller of the present disclosure according to an example embodiment is fixed and released.
FIG. 6 is a side cross-sectional view showing the state in which the fixing force for a ball part applied to a holding controller of the present disclosure according to an example embodiment is adjusted.
FIG. 7 is a side view showing the internal structure of a holding controller of the present disclosure according to another example embodiment.
FIG. 8 is a side view showing the internal structure of a holding controller of the present disclosure according to another example embodiment.

### Mode for Carrying Out the Invention

The present disclosure suggests a holding controller including a holding body having a ball part of a ball joint coupled to an object to be fixed, provided inside a front end, and configured to control a movement of the ball part, in which the holding body includes a fixing pressurization member configured to come into contact with the ball part, an elastic member configured to transmit force forward to the fixing pressurization member via elastic force, and a fixing controller configured to contract the elastic member backward or extend the elastic member forward, which is capable of adjusting a fixing force for holding an object to be fixed such as a medical device, thereby facilitating altering the position and angle of the object to be fixed in various situations.

The scope of the present disclosure is not limited to the example embodiments described below, and various modifications may be made by those skilled in the art without departing from the technical spirit of the present disclosure.

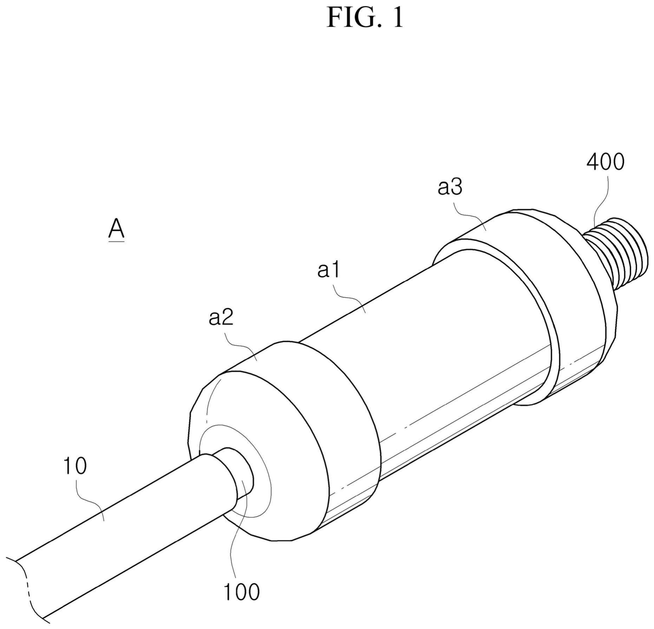

Hereinafter, a holding controller of the present disclosure will be described in detail with reference to accompanying FIGS. 1 to 8.

The holding controller of the present disclosure includes a holding body A for holding an object 10 to be fixed at a certain position and angle, or for altering the angle of the fixed object 10 at such a position, as shown in FIGS. 1 to 8. The object 10 to be fixed in the present disclosure may be a medical device, but is not limited thereto, and may be any other kind of device requiring the above operation.

The holding body A may have an exterior shape formed in the shape of a hollow rod. For example, as shown in FIGS. 1 to 4, it may include a case a1 that is open at both ends and hollow inside, a front cover a2 that is coupled to a front end of the case a1 and covers the front end of the case al, and a rear cover a3 that is coupled to a rear end of the case a1 and covers the rear end of the case a1.

The holding body A is provided with a ball joint 100 at the front end. The ball joint 100 may be part of the object 10 to be fixed or may be configured to allow the object 10 to be fixed to be coupled. The ball joint 100, which may be part of or coupled to the object 10 to be fixed, may include a ball part 110 forming a sphere shape and an arm part 120 forming a rod shape and extending from the ball part 110. The ball part 110 may be provided within the front end of the holding body A, and the arm part 120 may be provided to penetrate the front end of the holding body A.

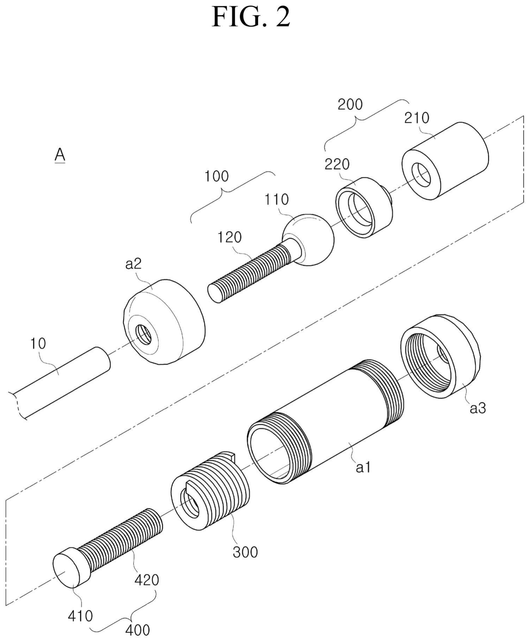

The holding body A includes, as shown in FIGS. 2 to 4, a fixing pressurization member 200, an elastic member 300, and a fixing controller 400 inside thereof in a configuration capable of controlling the movement of the ball part 110.

The fixing pressurization member 200 is provided at the rear of the ball part 110 so that it can contact the ball part 110, and can move within the holding body A according to the elastic force of the elastic member 300 and contact the ball part 110 when moving toward the front. In one example, the front end of the fixing pressurization member 200 that directly contacts the ball part 110 may be formed in the shape of a hemisphere so that the ball part 110 can be stably seated. In another example, as shown in FIGS. 2 to 4, the fixing pressurization member 200 may include a cylinder 210 formed in a "C" shape with an end face open toward the rear or in a tubular shape open toward the front and rear, and a ball casing 220 coupled to the front end of the cylinder 210 and having a hemispherical shape at the front end to receive a portion of the ball part 110.

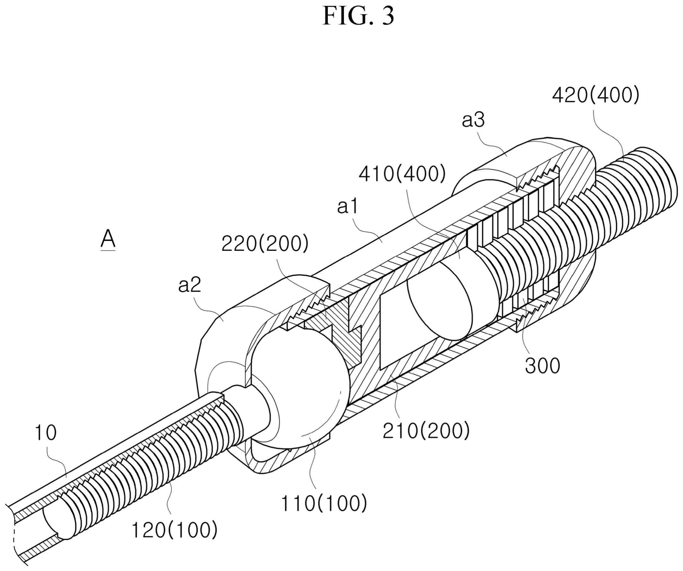

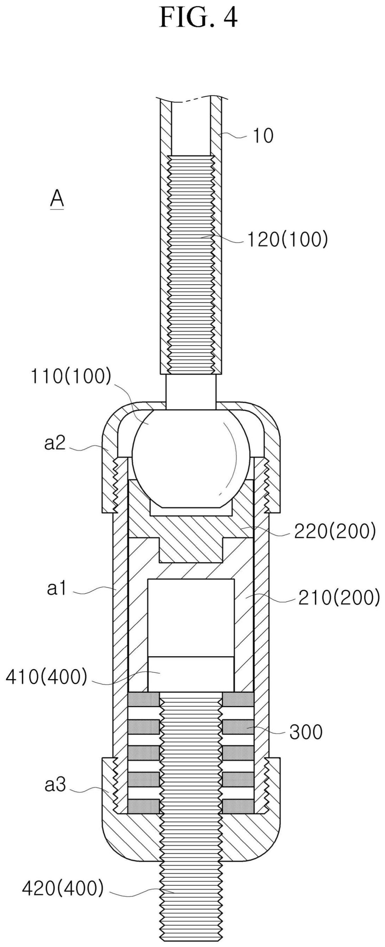

The elastic member 300, which is capable of moving the fixing pressurization member 200 forward through elastic force, may be formed of various types of springs. For example, as shown in FIGS. 3 and 4, the elastic member 300 may be formed of a coiled compression spring, configured with a front end abutting the cylinder 210 and a guard part 410 to be described later, and a rear end abutting the rear cover a3. In this case, the inner diameter of the rear end of the fixing pressurization member 200, that is, the rear end of the cylinder 210, is preferably formed larger than the inner diameter of the front end of the elastic member 300, and the diameter of the guard part 410 is preferably formed larger than the inner diameter of the front end of the elastic member 300.

The fixing controller 400 is configured to contract or extend the elastic member 300. In one example, the fixing controller 400 may include, as shown in FIGS. 2 to 4, the guard part 410 that is movable in a forward and backward direction within the cylinder 210 forming the fixing pressurization member 200 and abuts the front end of the elastic member 300, and a control rod 420 that extends rearward from the guard part 410 to penetrate the elastic member 300 and is screw-coupled to the rear end of the holding body A.

Specifically, the guard part 410 may be formed in the shape of a plate or barrel having a circular or polygonal cross-section, with an outer end configured to be positioned between the outer diameter and the inner diameter of the elastic member 300. The guard part 410 may be rotatable in contact with the front end of the elastic member 300, and a thrust bearing may be provided at the rear end to enable smooth rotation while reducing friction with the elastic member 300. The control rod 420 is formed in the shape of a rod having a diameter smaller than the inner diameter of the elastic member 300 and is threaded on the outer circumferential surface to be screw-coupled with the rear cover a3 constituting the rear end of the holding body A. Furthermore, the portion of the control rod 420 that is positioned on the outer side of the holding body A may be configured to interlock with the motor (not shown) for easy rotation of the control rod 420.

The fixing controller 400 may be axially rotated about the longitudinal axis of the control rod 420, which is an axis running in a forward and backward direction. As it rotates, the length of the elastic member 300 is adjusted and the fixing pressurization member 200 is moved, thereby fixing or releasing the ball part 110 or adjusting the fixing force on the ball part 110. In other words, the elastic member 300 may be contracted or extended depending on the direction in which the fixing controller 400 is axially rotating, and the fixing force of the ball part 110 may be adjusted by varying the extent to which the fixing controller 400 is axially rotated so that the fixing pressurization member 200 presses the ball part 110.

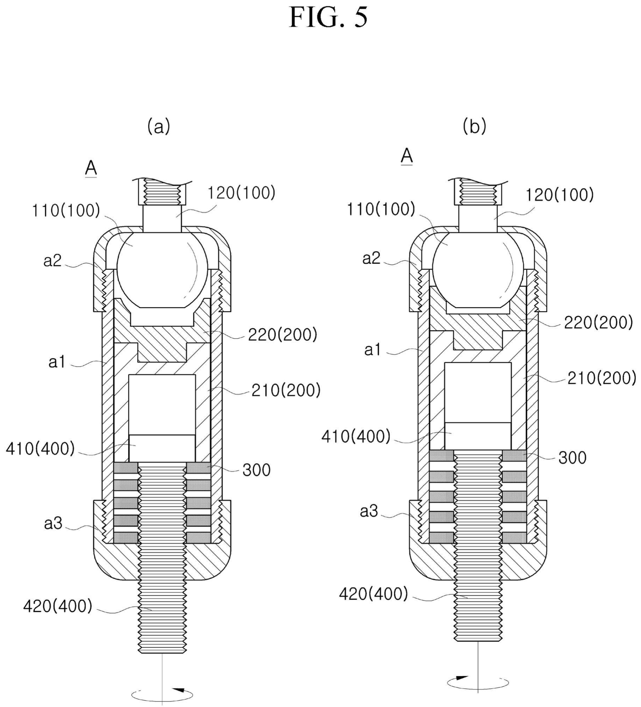

Specifically, when the control rod 420 is rotated in a direction moving toward the rear as shown in (a) of FIG. 5, the elastic member 300 is contracted by the guard part 410, which can cause the fixing pressurization member 200 to lose contact with the ball part 110. In this case, the ball part 110 is free to move, allowing the user to manipulate the fixed object 10 to achieve the desired position and angle.

With the contact between the fixing pressurization member 200 and the ball part 110 released as described above, rotation of the control rod 420, which is moved forward as shown in as shown in (b) of FIG. 5, causes the elastic member 300 to extend by the amount to which the guard part 410 is moved, and the force (elasticity) transmitted by the elastic member 300 causes the fixing pressurization member 200 to press the ball part 110 while engaging the ball part 110. In this case, the ball part 110 is restricted in movement, so that the fixed object 10 can be fixed at a specific position with a specific angle.

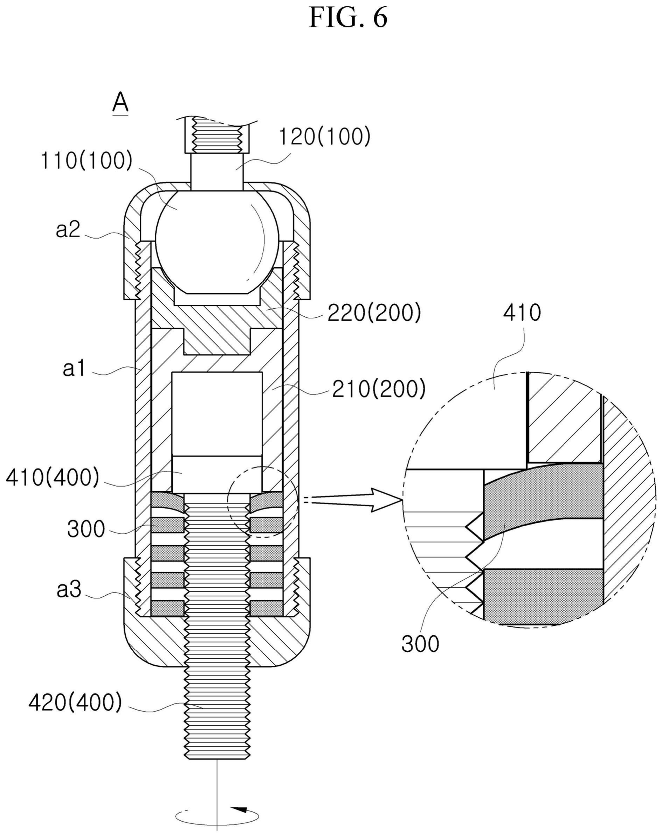

When the control rod 420 is slightly rotated in the direction moving toward the rear as shown in FIG. 6 while the elastic member 300 is in an extended state, the front end of the elastic member 300 having an outer diameter larger than the guard part 410 is bent by the downward force exerted by the guard part 410 being slightly moved. In this case, the position and angle of the fixed object 10 can be adjusted by moving the fixed object 10 in a state that the fixing force on the ball part 110 is somewhat reduced (e.g., 30%, 50%, 70%, etc. of the maximum fixing force).

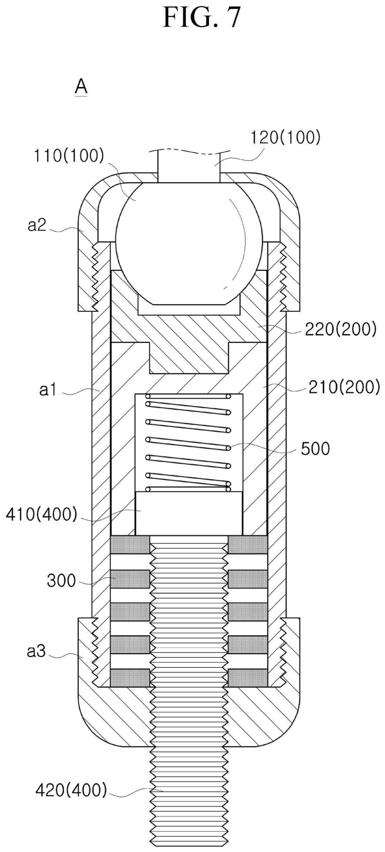

The holding body A may further include an auxiliary elastic member 500 that is provided in front of the fixing controller 400 inside the fixing pressurization member 200 and has elasticity. In one example, the auxiliary elastic member 500 may be formed of a compression spring having a smaller elastic force than the elastic member 500, as shown in FIG. 7, and may be positioned in front of the guard part 410 within the cylinder 210 and may be elastically actuated in a forward and backward direction. This auxiliary elastic member 500 prevents sudden release of fixation and facilitates fine control of the fixing force.

In other words, the auxiliary elastic member 500 is in a state that the front end is elastically supported on the cylinder 210 and the rear end is supported on the guard part 410. Accordingly, the ball part 110 is not abruptly released from fixation even if the control rod 420 is rotated in a direction that the elastic member 300 is moved toward the rear in an extended state since the auxiliary elastic member 500 pushes the cylinder 210 with an elastic force, and fine adjustment of the fixing force can be made more smoothly. Furthermore, due to the auxiliary elastic member 500, the fixing force adjustment of the ball part 100 can be made regardless of the direction of the front end of the holding body A with the ball joint 100.

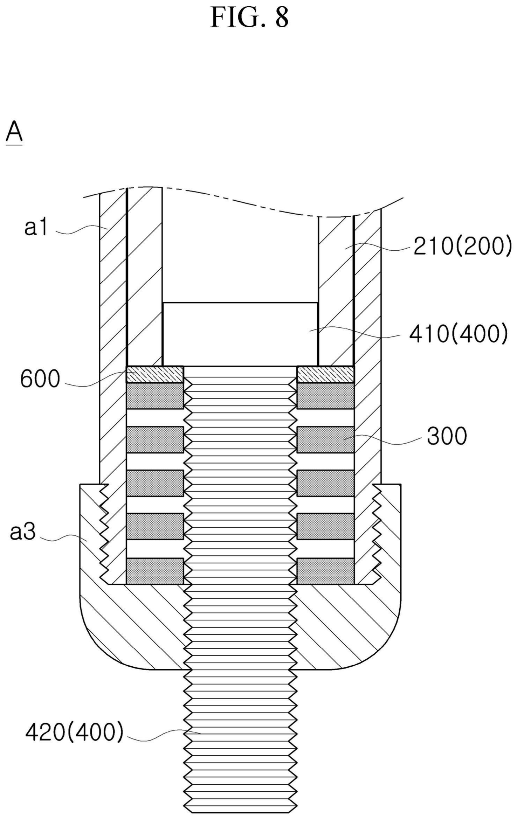

Further, the holding body A may further include an adjustment guide plate 600 provided at the front end of the elastic member 300, on which the rear end of the fixing pressurization member 200 and the guard part 410 abut. In one example, the adjustment guide plate 600 may be formed in a ring shape to correspond to a cross-section of the elastic member 200, as shown in FIG. 8, and may bend upon fine adjustment of the fixing force. This is to prevent the elastic force from being affected when the front end of the elastic member 400 comes into direct contact with the guard part 410 and continuous bending occurs.

**Description of Reference Numerals**

| | | | |
|---|---|---|---|
| A: | Holding body | a1 : | Case |
| a2 : | Front cover | a2 : | Rear cover |
| 10 : | Object to be fixed | | |
| 100 : | Ball joint | 110 : | Ball part |
| 120 : | Arm part | | |
| 200 : | Fixing pressurization member | 210 : | Cylinder |
| 220 : | Ball casing | | |
| 300 : | Elastic member | | |
| 400 : | Fixing controller | 410 : | Guard part |
| 420 : | Control rod | | |
| 500 : | Auxiliary elastic member | | |
| 600 : | Adjustment guide plate | | |

## Claims

1. A holding controller comprising:
a holding body (A) having a ball part (110) of a ball joint (100) coupled to an object (10) to be fixed, provided inside a front end, and configured to control a movement of the ball part (110), wherein
the holding body (A) comprises a fixing pressurization member (200) configured to come into contact with the ball part (110), an elastic member (300) configured to transmit force forward to the fixing pressurization member (200) via elastic force, and a fixing controller (400) configured to contract the elastic member (300) backward or extend the elastic member (300) forward.

2. The holding controller of claim 1, wherein
the fixing controller (400) is configured to axially rotate about an axis oriented in a forward and backward direction, and
an extent to which the fixing pressurization member (200) pressurizes the ball part (110) changes depending on contraction or extension of the elastic member (300) according to the direction of axial rotation of the fixing controller (400).

3. The holding controller of claim 2, wherein the fixing controller (400) comprises a guard part (410) configured to move forward and backward within the fixing pressurization member (200) and contract the elastic member (300) and a control rod (420) extending rearward from the guard part (410) to penetrate the elastic member (300) and being screw-coupled to a rear end of the holding body (A).

4. The holding controller of claim 3, wherein
the elastic member (300) is formed of a compression spring, and
an outer end of the guard part (410) is positioned between an outer diameter and an inner diameter of the elastic member (300).

5. The holding controller of claim 3, further comprising an adjustment guide plate (600) provided at a front end of the elastic member (300) on which a rear end of the fixing pressurization member (200) and the guard part (410) abut.

6. The holding controller of claim 1, wherein the holding body (A) further comprises an auxiliary elastic member (500) having elasticity and provided in front of the fixing controller (400) inside the fixing pressurization member (200).
